# EUROPEAN PATENT SPECIFICATION

(11) **EP 2 153 809 B1**
(45) Date of publication and mention of the grant of the patent: **09.12.2015**
(21) Application number: 08162068.4
(22) Date of filing: 08.08.2008
(51) Int. Cl.: A61F 13/532, A61F 13/533, A61L 15/18, A61F 13/535, A61F 13/537

(54) **Absorbent core**
Saugfähiger Kern
Noyau absorbant

(43) Date of publication of application: 17.02.2010
(73) Proprietor: The Procter & Gamble Company, Cincinnati, OH 45202 (US)
(72) Inventor: Carlucci, Giovanni, 66100, Chieti (IT); Tamburro, Maurizio, 66020, Sambuceto (Chieti) (IT); Toro, Evelina, 66100, Chieti (IT)
(74) Representative: Briatore, Andrea

(56) References cited:
- WO-A-95/26209
- GB-A- 2 283 679

## Description

### FIELD OF THE INVENTION

The present invention relates to an absorbent core for absorbent articles, for example sanitary napkins and the like.

### BACKGROUND OF THE INVENTION

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core can typically comprise one or more fibrous absorbent material, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure. Absorbent gelling materials can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure can further provide an improved immobilization of absorbent gelling materials, particularly when the article is fully or partially loaded with liquid, and an increased wearing comfort. Such thinner structures provide absorbent articles combining better comfort, discreetness and adaptability, such as for example, thin absorbent structures where the absorbent gelling material is located and somehow kept in selected, e.g. patterned regions of the structure itself.

EP 1447067, assigned to the Procter & Gamble Company, describes an absorbent article, typically a disposable absorbent article, such as a diaper, having an absorbent core which imparts increased wearing comfort to the article and makes it thin and dry. The absorbent core comprises a substrate layer, the substrate layer comprising a first surface and a second surface, the absorbent core further comprising a discontinuous layer of absorbent material, the absorbent material comprising an absorbent polymer material, the absorbent material optionally comprising an absorbent fibrous material which does not represent more than 20 weight percent of the total weight of the absorbent polymer material. The discontinuous layer of absorbent material comprises a first surface and a second surface, the absorbent core further comprising a layer of thermoplastic material, the layer of thermoplastic material comprising a first surface and a second surface and wherein the second surface of the discontinuous layer of absorbent material is in at least partial contact with the first surface of the substrate layer and wherein portions of the second surface of the layer of thermoplastic material are in direct contact with the first surface of the substrate layer and portions of the second surface of the layer of thermoplastic material are in direct contact with the first surface of the discontinuous layer of absorbent material.

While absorbent articles according to EP 1447067 and comprising thin absorbent cores with relatively high amounts of absorbent gelling materials and rather low content of fibrous materials commonly have good absorption and retention characteristics to body fluids like urine, there still remains room for improvement of absorption and retention, particularly towards other body fluids. In particular, menses, blood and vaginal discharges are particularly difficult to be effectively absorbed and retained into absorbent cores containing superabsorbent materials in major amounts since such materials may not show optimal absorption and retention characteristics towards such body fluids. Particularly, superabsorbent materials may show a relatively slow acquisition and absorption rate.

WO 95/26209 A1 describes an absorbent member comprising AGM and intert materials.

It is believed that the non-optimal absorption and retention are mainly caused by poor permeability of superabsorbent materials towards menses, blood or vaginal discharges due to the viscosity and/or to the complex nature of these fluids. For example menses and blood are water based fluids comprising components having molecular weights higher than water and also corpuscular components, including red cells, white cells, soluble proteins, cellular debris and mucus, which slow down the absorption of these fluids by superabsorbents. Menses and blood are rather thick, and more difficult to absorb in conventional absorbent structures comprising absorbent gelling materials; moreover, corpuscular components like red cells may decrease the absorption capacity of certain superabsorbent particles. This adverse effect can also be enhanced by the fact the absorbent gelling material, usually in form of particles, can be provided in relatively high amount and concentration, typically with particles in close contact to one another, at least in some regions of the absorbent core, which upon absorption of fluid and subsequent swelling might involve a reduced permeability of the layer of absorbent material. This translates into a slower initial uptake rate of the fluid into the superabsorbent material, and in turn in the absorbent structure comprising the superabsorbent material, which can result in a lower final absorption and retention capacity.

The present invention provides significant improvements in the above area by the incorporation of an inert material in an absorbent core structure for an absorbent article, particularly for absorption of menses or blood or vaginal discharges, which comprises the absorbent gelling material in a non uniform layer stably provided onto a fibrous substrate layer.

### SUMMARY OF THE INVENTION

The present invention addresses the above needs by providing an absorbent core for an absorbent article intended for absorption of menses or blood or vaginal discharges, according to the claims.

### BRIEF DESCRIPTION OF THE DRAWINGS

Figure 1 is a plan view of a sanitary napkin showing an absorbent core according to an embodiment of the present invention, with portions of some constituent elements cut out in order to show underlying elements.
Figure 2 is a schematic cross section of the sanitary napkin of Figure 1 taken in the transverse axis A-A'.
Figure 3 shows a schematic cross section of an absorbent core according to one embodiment of the present invention.
Figure 4 shows a schematic cross section of an absorbent core according to another embodiment of the present invention.
Figure 5 shows a perspective view of an exemplary absorbent core according to the present invention.

### DETAILED DESCRIPTION OF THE INVENTION

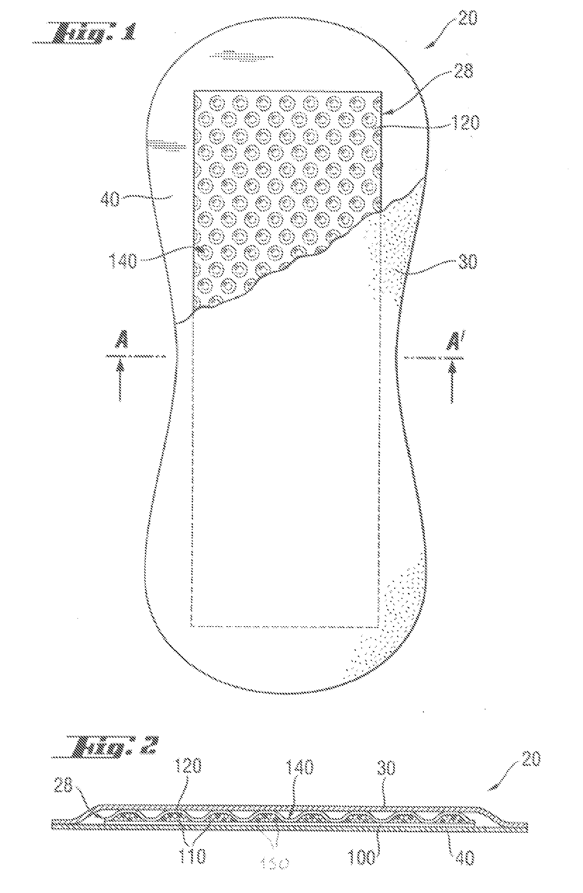

The present invention relates to an absorbent core for absorbent articles such as sanitary napkins, panty liners, tampons, interlabial devices, wound dressings, and the like, which are intended for the absorption of body fluids, such as menses or blood or vaginal discharges. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

### Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

### Absorbent Core

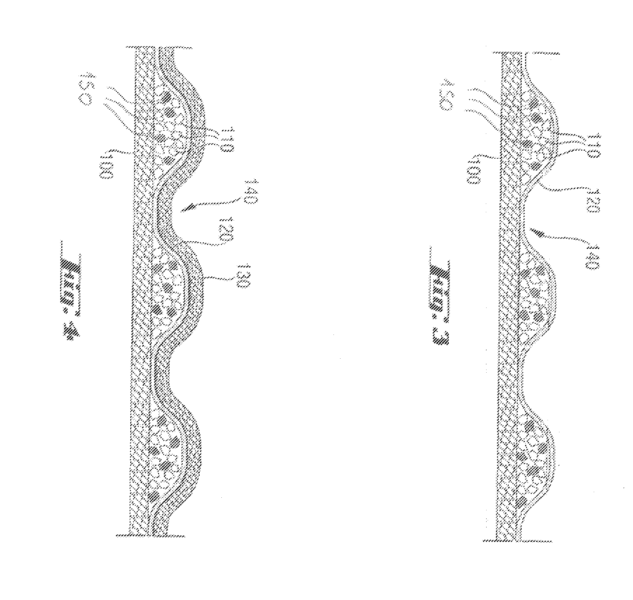

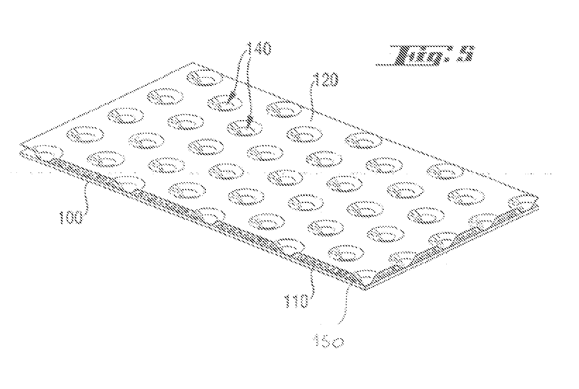

According to the present invention, and as shown for example in the embodiments of Figures 3 and 5, the absorbent core 28 can comprise a substrate layer 100, absorbent polymer material 110, a layer of thermoplastic material 120, which is a layer of fiberized hot melt adhesive 120. The substrate layer 100 can be typically provided from a fibrous material, as will be explained in detail below.

An alternative embodiment of the present invention is shown in Figure 4. The absorbent core shown in Figure 4 can further comprise a cover layer 130. This cover layer may be provided of the same material as the substrate layer 100, or may be provided from a different material. Suitable materials for the cover layer are for example nonwoven materials, as will be better explained further on.

The substrate layer 100 comprises a first surface and a second surface. Conventionally, in the sectional views illustrated in the attached drawings the first surface of each layer can be said to correspond to the top surface, in turn the wearer facing surface of the article 20, while the second surface corresponds to the bottom surface, in turn the garment facing surface. At least portions of the first surface of the substrate layer 100 are in contact with a layer of absorbent polymer material 110. This layer of absorbent polymer material 110 can be typically a non uniform layer, and comprises a first surface and a second surface, wherein by "non uniform" it is meant that the absorbent polymer material 110 is distributed over the substrate layer 100 with non uniform basis weight. Conversely, the second surface of the non uniform layer of absorbent polymer material 110 is in at least partial contact with the first surface of the substrate layer 100. According to an embodiment of the present invention, the non uniform layer of absorbent polymer material 110 can be a discontinuous layer that is a layer typically comprising openings, i.e. areas substantially free of absorbent polymer material, which in certain embodiments can be typically completely surrounded by areas comprising absorbent polymer material, as will be explained in more detail later on. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 are in contact with at least portions of the first surface of the substrate layer material 100. The first surface of the absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 are not covered by absorbent polymer material 110. The absorbent core 28 further comprises a layer of a thermoplastic material 120. This thermoplastic material 120 serves to at least partially immobilize the absorbent polymer material 110.

In a typical embodiment of the present invention the thermoplastic material 120 can be provided as a fibrous layer which is partially in contact with the absorbent polymer material 110 and partially in contact with the substrate layer 100. Figures 3 and 5 show such a structure in an exemplary embodiment of the present invention. In this structure the absorbent polymer material layer 110 is provided as a discontinuous layer, a layer of fiberized thermoplastic material 120 is laid down onto the layer of absorbent polymeric material 110, such that the thermoplastic layer 120 is in direct contact with the first surface of the layer of absorbent polymer material 110, but also in direct contact with the first surface of the substrate layer 100, where the substrate layer is not covered by the absorbent polymeric material 110, i.e. typically in correspondence of the openings of the discontinuous layer of the polymer material 120. By "direct contact" it is meant that there is no further intermediate component layer between the layer of thermoplastic material 120 and the other respective layer in direct contact thereto, such as for example a further fibrous layer. It is however not excluded that a further adhesive material can be comprised between the layer of thermoplastic material 120 and the optional cover layer 130, when present, as shown in Figure 4, or the layer of absorbent polymer material 110 or, more typically, the substrate layer 100, such as for example a supplementary adhesive material provided onto the first surface of the substrate layer 100 to further stabilize the overlying absorbent polymer material 110. "Direct contact" can hence be considered to mean in this context a direct adhesive contact between the layer of thermoplastic material 120 and the other respective layer as explained above. This imparts an essentially three-dimensional structure to the fibrous layer of thermoplastic material 120 which in itself is essentially a two-dimensional structure of relatively small thickness (in z-direction), as compared to the extension in x- and y-direction. In other words, the fibrous thermoplastic material layer 120 undulates between the first surface of the absorbent polymer material 110 and the first surface of the substrate layer 100. The areas where the fibrous thermoplastic material 120 is in contact with the substrate layer 100 are the areas of junction 140.

Thereby, the thermoplastic material 120 provides spaces to hold the absorbent polymer material 110 typically towards the substrate layer 100, and thereby immobilizes this material. In a further aspect, the thermoplastic material 120 bonds to the substrate 100 and thus affixes the absorbent polymer material 110 to the substrate 100. Typical thermoplastic materials will also penetrate into both the absorbent polymer material 110 and the substrate layer 100, thus providing for further immobilization and affixation.

In the alternative embodiment representatively illustrated in Figure 4 portions of the cover layer 130 bond to portions of the substrate layer 100 via the thermoplastic material 120. Thereby, the substrate layer 100 together with the cover layer 130 provides spaces to immobilize the absorbent polymer material 110.

Of course, while the thermoplastic materials disclosed herein can provide a much improved wet immobilisation, i.e. immobilisation of absorbent polymer material when the article is wet or at least partially loaded, these thermoplastic materials can also provide a very good immobilisation of absorbent polymer material when the article is dry.

In accordance with an embodiment of the present invention, the absorbent polymer material 110 may also be optionally mixed with fibrous material, which can provide a matrix for further immobilization of the absorbent polymer material. However, typically a relatively low amount of fibrous material can be used, for example less than 40 weight %, less than 20 weight %, or less than 10 weight % of the total weight of the absorbent polymer material 110, positioned within the areas of absorbent polymer material.

According to an embodiment of the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material, as shown for example in Figure 5. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of thermoplastic material 120. Hence, in summary, a discontinuous layer of absorbent polymer material 110 may comprise connected areas of absorbent polymer material 110, as e.g. illustrated in Figure 5, or may alternatively comprise discrete areas of absorbent polymer material 110.

The present invention, and specifically the embodiments described with reference to Figures 3, 4 and 5 can be used to provide a storage layer of an absorbent core. However, they can also be used to provide the full absorbent core 28 as illustrated in Figure 1. In that case, no further materials wrapping the core, such as for example a top layer and a bottom layer are being used. With reference to the embodiments of Figure 4 the optional cover layer 130 may provide the function of a top layer and the substrate layer 100 may provide the function of a bottom layer of an absorbent core, wherein top and bottom layers respectively correspond to the body facing and garment facing surfaces of the core 28.

With reference to Figures 3, 4 and 5 the areas of direct contact between the thermoplastic material 120 and the substrate material 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140 can be disposed in a regular or irregular pattern. For example, the areas of junction 140 may be disposed along lines as shown in Figure 5. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence for example the areas of junction 140 can be arranged along lines which form an angle of 20 degrees, or 30 degrees, or 40 degrees, or 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the thermoplastic layer 120 can comprise any thermoplastic material, and typically adhesive thermoplastic materials, also referred to as hot melt adhesives. A variety of thermoplastic materials can be suitable to immobilize the absorbent polymer material. Some initially thermoplastic materials may later lose their thermoplasticity due to a curing step, e.g. initiated via heat, UV radiation, electron beam exposure or moisture or other means of curing, leading to the irreversible formation of a crosslinked network of covalent bonds. Those materials having lost their initial thermoplastic behaviour can be herein also understood as thermoplastic materials 120.

Without wishing to be bound by theory it has been found that those thermoplastic materials, i.e. typically the hot melt adhesives, can be most useful for immobilizing the absorbent polymer material 110, which combine good cohesion and good adhesion behaviour. Good adhesion is critical to ensure that the thermoplastic layer 120 maintains good contact with the absorbent polymer material 110 and in particular with the substrate material 100. Good adhesion is a challenge, namely when a non-woven substrate material is used. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive not break, which would deteriorate the wet immobilization. Exemplary suitable thermoplastic materials can be as described in the already mentioned patent application EP 1447067, particularly at sections [0050] to [0063].

The thermoplastic material, typically a hotmelt adhesive, can be present in the form of fibres throughout the core, being provided with known means, i.e. the adhesive can be fiberized. Typically, the fibres can have an average thickness of 1 - 100 micrometer and an average length of 5 mm to 50 cm. In particular the layer of thermoplastic material, typically e.g. a hot melt adhesive, can be provided such as to comprise a net-like structure.

To improve the adhesiveness of the thermoplastic material 120 to the substrate layer 100 or to any other layer, in particular any other non-woven layer, such layers may be pre-treated with an auxiliary adhesive.

In particular, typical parameters of a hot melt adhesive in accordance with the present invention can be as follows.

In an aspect, the loss angle tan Delta of the adhesive at 60°C should be below the value of 1, or below the value of 0.5. The loss angle tan Delta at 60°C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, hot melt adhesives in accordance with the present invention may have a sufficient cohesive strength parameter γ. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

In the absorbent core of the present invention the substrate layer 100 and the optional cover layer 130 can be typically provided from nonwoven materials, for example spunbonded or carded nonwoven materials, or also airlaid materials, such as for example latex and/or thermal bonded airlaid materials.

Exemplary materials for the substrate layer 100 can comprise fibrous materials comprising cellulose fibres, typically not more than 60% by weight of cellulose fibres, or from 30% to 50% by weight of cellulose fibres. Examples of fibrous materials for the substrate layer 100 can be nonwoven materials, such as for example carded nonwovens, or more typically airlaid or wetlaid fibrous materials, such as for example latex or thermal bonded airlaid fibrous materials, comprising synthetic and natural fibres, such as for example cellulose fibres. Basis weights for the materials of the substrate layer 100 can typically range from 10 g/m² to 120 g/m², or from 40 g/m² to 100 g/m², or also from 50 g/m² to 80 g/m².

Exemplary materials for the optional cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovens. Other nonwoven materials for the optional cover layer 130 can comprise composite structures such as a so called SMS material, comprising a spunbonded, a melt-blown and a further spunbonded layer. Basis weights for the materials of the cover layer 130 can typically range from 5 g/m² to 80 g/m², or from 10 g/m² to 60 g/m², or also from 20 g/m² to 40 g/m²

In certain embodiments of the present invention the absorbent polymer material 110 in the absorbent core 28 is present throughout the area of the absorbent core in an average basis weight of less than 250 g/m², or of less than 220 g/m², or from 60 g/m² to 180 g/m², or from100g/m² to 160 g/m². An average basis weight is typically based on the whole area of the zone of application, i.e. interested by the layer of absorbent polymer material, and hence comprising possible openings included in an e.g. discontinuous layer. Typically, the absorbent polymer material 110 can constitute at least 45%, or at least 50%, or at least 55%, by weight of the absorbent core, wherein the absorbent core can typically correspond to the embodiments described with reference to Figures 3, 4, and 5, hence comprising the substrate layer, the layer of absorbent polymer material, the layer of thermoplastic material, the optional cover layer if present, and any other material possibly comprised within this structure, such as for example the additional fibrous material mentioned above, additional adhesive material, but excluding the inert material.

Typically the absorbent polymer material for the absorbent cores according to the present invention can comprise absorbent polymer particles having a selected average particle size.

According to the present invention, the absorbent core further comprises an inert material 150, or a mixture of inert materials.

By "inert material", it is herein meant a material, typically in particulate form, which is inert towards the fluids which can be typically absorbed by the absorbent core of the present invention, i.e. it does not react with them nor swells upon contact, although it can be wetted, and possibly show some minor absorption of fluid, for example within pores. Suitable inert materials can typically comprise an inorganic powder or particulate material, and can include, but are not limited to, silica, silicon dioxide, amorphous silica, alumina, titanium dioxide, clays such as Kaolin and Montmorillonite clays, or also the inorganic materials described in patent US 4,500,670 as inorganic powders. Silica gel can also be used. Typically, the inert material used in the present invention can comprise silica, silicon dioxide, amorphous silica, or silica gel. Silica and silica gel can be typically used.

The inert material in particle form 150, or the mixture of inert materials, is provided in a process step directly to the non uniform layer of particulate absorbent gelling material, for example intermixed with it in the same lay-down system. Typically the inert material and the absorbent gelling material, both in particulate form, can be homogeneously mixed together before being provided onto the substrate layer in order to form the non uniform layer of absorbent polymer material, which hence also comprises the inert material thoroughly mixed therein.

According to an embodiment of the present invention, the average particle size of the particulate absorbent polymer material and of the inert material in particulate form can be suitably selected.

The average particle size of a material in particulate form, namely for example the absorbent polymer material and the inert material, can be determined as it is known in the art, for example by means of dry sieve analysis. Optical methods, e.g. based on light scattering and image analysis techniques, can also be used. A method is described hereinafter in the Test Methods section.

In the absorbent core of the present invention the non uniform layer of absorbent polymer material 110 can at most typically comprise a relatively low amount of fibrous material, or possibly none at all, as explained above, hence all or nearly all absorbent capacity in the absorbent core of the present invention is typically provided by the absorbent polymer material 110 comprised in the non uniform layer. While the absorbent polymer material can typically have a high absorption capacity, it can show a rather slow acquisition capacity and absorption rate, particularly towards complex body fluids such as menses or blood or vaginal discharges. The absorbent polymer material 110 in fact, typically in form of particles, can be typically provided in the non uniform layer in relatively high amount and concentration, with the particles closely packed to one another, at least in certain areas of the layer, as can be seen in Figures 3, 4, and 5. Upon fluid absorption, and subsequent swelling of the absorbent polymer material particles, further fluid acquisition and absorption within and through the at least partially swollen absorbent polymer material can be slowed down, at least to a certain extent.

The inert material in particulate form, typically homogeneously mixed among the absorbent polymer material particles 110 forming the non uniform layer, can provide for a better handling of the fluid, by spacing the absorbent polymer material particles and hence typically increasing the contact surface between the absorbent polymer material and the fluid. This can translate into a better fluid handling and acquisition, particularly towards further fluid amounts contacting the non uniform layer of the absorbent core of the present invention. In addition, this can also increase the permeability of the non uniform layer of absorbent polymer material towards fluid, in turn allowing for example to take better advantage of the absorbent properties of e.g. the substrate layer 100, which can be more easily reached by fluid through the non uniform layer of absorbent polymer material. This can typically reduce or eliminate the risk of fluid leakage or rewetting, which could in principle be caused by fluid still "free" within the structure of an absorbent core similar to that of the present invention, i.e. typically thin and usually free, or with only a relatively minor amount, of fibrous material specifically meant for fluid absorption, and without the inert material, during the relatively slow absorption of the fluid by the absorbent polymer material 110.

According to an embodiment of the present invention, the average particle size, defined as explained herein, of the inert material, typically in particulate form, can be selected in order to be similar to the average particle size of the absorbent polymer material, also typically in particulate form. For example, the average particle size of the absorbent polymer material can be from 200 µ to 600 µ, or from 300 µ to 500 µ, wherein the average particle size of the inert material can also be from 200 µ to 600 µ, or from 300 µ to 500 µ. In an alternate embodiment of the present invention, the average particle size of the inert material can be from 65% to 130%, or from 70% to 110%, of the average particle size of the absorbent polymer material, wherein the average particle size of the two materials can be usually measured with the same method and under the same conditions.

In an embodiment of the present invention, the bulk density of the absorbent polymer material and of the inert material, both typically in particle form, can be also suitably selected. The bulk density of a material in powder or particulate form, also known as apparent density, refers to the weight per unit volume of the material, including voids inherent in the material. It can be measured according to standard methods known in the art; for example, in the context of the present invention, the bulk/apparent density can be measured according to the method referred to hereinafter, in the Test Methods section.

Typically the bulk density of the absorbent polymer material can be from about 0.5 g/cm³ to about 0.9 g/cm³. According to an embodiment of the present invention, the bulk density of the inert material can be from 70% to 120%, or from 80% to 110% the bulk density of the absorbent polymer material.

The amount of the inert material in an embodiment of the present invention can constitute from 10% to 100%, or from 20% to 70%, or also from 30% to 60% by weight of the amount of absorbent polymer material.

The selection of the average particle size and/or of the bulk density of the inert material, typically with reference to the respective average particle size and bulk density of the absorbent polymer material, as considered individually or alternatively in combination, can provide for a more effective mixing of the two materials, both typically in particle form, within the layer of absorbent polymer material in the absorbent core of the present invention, particularly in a more uniform mixture also in presence of relatively high amounts of inert material. This can translate into an even better fluid handling, as outlined above, particularly towards complex body fluids such as menses or blood or vaginal discharges.

Additionally, handling of the absorbent polymer material and of the inert material, both typically in particle form, for example in a production line for the manufacturing of an absorbent core according to an embodiment of the present invention, can be facilitated when the respective average particle size and/or the bulk density are selected as explained above. Provision of a non uniform layer of absorbent polymer material in the desired arrangement, also comprising the inert material in the selected amount homogeneously mixed therein, as previously described, can hence be achieved more effectively with usual dosing and mixing apparatuses in the manufacturing line.

In certain embodiments of the absorbent core 28 of the present invention the inert material 150, or the mixture of inert materials, can be present in the absorbent core in an average basis weight of from 10 g/m² to 250 g/m², or from 20 g/m² to 100 g/m², or from 40 g/m² to 70 g/m², by weight of the inert material per square meter of the zone of application. An average basis weight is therefore typically based on the area actually interested by the application of the inert material. Typically the areas interested by the application of the absorbent polymer material and of the inert material can coincide, as the two materials can be typically homogeneously mixed.

According to the present invention, the absorbent core can provide a more efficient fluid management, in terms of acquisition, immobilization and absorption, as explained above, which can be particularly useful in case of complex body fluids such as menses or blood. Overall, this increased efficiency in the composite structure according to the present invention can translate in a more effective exploitation of the absorbent capacity of the absorbent polymer material, also in presence of problematic body fluids such as menses or blood or vaginal discharges, and possibly also in a more efficient use of the entire structure of the absorbent core, for example taking advantage of the absorbent capacity of the substrate layer, as explained above.

This is achieved in a structure which is typically thin and is capable of employing more completely the absorption and immobilization capacity of the different materials, particularly the absorbent polymer material which can hence be present in a typically lesser amount, in synergy with the inert material or materials, thus overall also providing a particularly thin structure having improved dimensional stability during absorption and therefore increased comfort during use.

According to an embodiment of the present invention the absorbent polymer material can be selected among the polyacrylate based polymers described in the PCT Patent Application WO2007/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between 30% and 80% by weight, or between 32% and 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The absorbent polymer material can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional superabsorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core. Synergy with inert material can be particularly effective with said absorbent polymer materials.

According to the present invention, the absorbent core 28 can fully constitute the absorbent element in an absorbent article, or can constitute part of it, being complemented with other layers in a composite structure. Also, an absorbent article comprising an absorbent core according to the present invention can further comprise a fibrous acquisition layer between the absorbent core 28 and the topsheet. According to an embodiment of the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from 10 g/m² to 60 g/m², or from 25 g/m² to 40 g/m².

According to another alternative embodiment of the present invention the absorbent article can comprise a further fibrous layer comprised between the absorbent core 28 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid which might not be fully retained by the absorbent core 28. The presence of cellulose fibres can make the layer particularly effective in acquiring and diffusing the fraction of body fluids like menses or blood which is not completely absorbed by the absorbent polymer material of the absorbent core 28.

An exemplary process for producing absorbent cores 28 in accordance with the present invention can comprise the following steps.

In one step, the substrate layer 100 is laid onto a formation surface. The absorbent polymer material 110 and the inert material 150, both typically in particulate form, can be homogeneously mixed together and are disposed by means known in the art, for example by means of a lay-down drum, in the selected non uniform e.g. discontinuous layer onto the substrate layer 100, optionally after providing a stabilizing adhesive on the substrate layer 100, for example in longitudinal stripes. In a further process step, a hot melt adhesive can be placed with known means onto the absorbent polymer material, for example in form of fibres.

While any adhesive application means known in the art can be used to place the hot melt adhesive onto the absorbent polymer material, the hot melt adhesive can be typically applied by a nozzle system. For example, a nozzle system can be utilised, which can provide a relatively thin but wide curtain of adhesive, for example in form of fibres. This curtain of adhesive is than placed onto the substrate layer 100 and the absorbent polymer material 110.

In a further process step, an optional cover layer 130 can be typically placed upon the substrate layer 100, the absorbent polymer material and the hot melt adhesive layer. The cover layer 130 will be in adhesive contact with the substrate layer 100 in the areas of junction 140. In these areas of junction 140 the adhesive is in direct contact with the substrate layer 100. The cover layer 130 will typically not be in direct adhesive contact with the substrate layer 100 where the absorbent polymer material 110 is present.

In one alternative embodiment, the cover layer 130 and the substrate layer 100 can be provided from a unitary sheet of material. The placing of the cover layer 130 onto the substrate layer 100 can then involve the folding of the unitary piece of material.

Hence, the uneven service of the lay-down system, which may be a lay-down drum, typically determines the distribution of absorbent polymer material and inert material in the non uniform, for example discontinuous layer and likewise can determine the pattern of areas of junction 140. The distribution of absorbent polymer material and inert material may be influenced by vacuum means.

The distribution of absorbent polymer material and inert material can be profiled, for example profiled in the longitudinal direction, or in the lateral direction, or in both, for example being substantially absent in an area along the longitudinal side ends of the absorbent core.

The absorbent polymer material for the absorbent cores according to the present invention, typically comprising absorbent polymer particles, according to a further embodiment of the present invention, can have a permeability, as expressed by the saline flow conductivity of the absorbent polymer material, greater than 10, 20, 30 or 40 SFC- units, where 1 SFC unit is 1 x 10⁻⁷ (cm³ x s) / g. Saline flow conductivity is a parameter well recognised in the art and is to be measured in accordance with the test disclosed in EP 752 892 B.

### Backsheet

The absorbent article comprising the core according to the present invention can also comprise a backsheet 40. The backsheet primarily has to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

### Example

A sanitary napkin comprising an absorbent core according to an embodiment of the present invention is similar to that illustrated in Figures 1 and 2 and comprises a topsheet constituted by a polyethylene perforated formed film, a backsheet constituted by a 25 g/m² polyethylene film, a core comprising a cover layer constituted by a 30 g/m² carded nonwoven comprising polyester fibres and PP/PE bicomponent fibres, available from BBA Fiberweb under the code TBPL 50/50 6dpf philic PET/BICO, a discontinuous layer of an absorbent polymer material constituted by a particulate superabsorbent material available from Nippon Shokubai under the trade name Aqualic L520, and of a silica gel available from W.R. Grace under the code SG 122 distributed onto the substrate layer in a non uniform layer having overall an average basis weight of 182 g/m², comprising 122 g/m² of the absorbent polymer material, and 60 g/m² of the inert material, and a layer of thermoplastic material constituted by a hot melt adhesive available from HB Fuller under the trade name NV 1151 Zeropack applied in fibres having an average thickness of about 50 µm at a basis weight of 11 g/m². The average particle size of the absorbent polymer material and of the inert material are respectively of 400 µ and 300 µ, while the bulk density is of 0.65 g/cm³ for the absorbent polymer material and of 0.70 g/cm³ for the inert material. The absorbent core further comprises a substrate layer, constituted by a 65 g/m² latex bonded airlaid (LBAL) material comprising 30% by weight cellulose fibres, 40% by weight PET fibres and 30% by weight latex binder, available from Concert GmbH under the code WHXX65.

### Test Methods

### Average particle size

The method is similar to that described in patent US 6,096,299 at lines 1-35 of column 3 can be used, slightly modified as explained below.

The average particle size of a material can be determined by a mechanical sieve shaker method using an electromagnetic sieve shaker Fritsch ® Analysette 3, or equivalent, with six Tyler Screen

Standard analytical sieves (20, 32, 42, 65, 100 and 150 Mesh, respectively corresponding to 841, 500, 354, 210, 149 and 105 µm) and one sieve pan. The selected sieves are nested with the coarsest sieve at the top and the solid pan at the bottom, then the test sample (100 g of material) is placed on the top sieve and the nest is closed with a cover. The shaker is operated for 15 minutes continuously with an amplitude of vibrations of 1 mm and finally, after the completion of the agitation, the material retained on each sieve is weighed separately. The material which has passed through the finest sieve into the pan is also weighed.

These weights and the weight of the original test sample are used to calculate the average particle size of the test sample. In order to get the average particle size the percentage retained on each sieve is calculated by dividing the "total weight coarser" than that sieve by the total weight of the test sample. The total weight coarser includes the material retained on that particular sieve plus all material on all coarser sieves. This cumulative percentage represents the total percentage of the test sample coarser than the aperture of that particular sieve.

The data is plotted on a sieve analysis graph where the abscissa represents the sieve sizes (on a logarithmic scale) and the ordinate the percentages retained (on a linear scale). By interpolation on the sieve analysis graph the sieve size corresponding to a percentage of 50% retained can be evaluated, and this size is taken as the average particle size of the sample.

### Apparent density

The apparent density, also known as bulk density, of the absorbent polymer material and of the inert material, both typically in particle form, can be measured according to the Edana Standard Test WSP 260.2 (05).

It has to be noted that both the test for the average particle size and the test for the apparent/bulk density shall be conducted in a controlled environment, typically at 23±2°C and 50±10% RH. The sample material for both tests shall be dried in an oven for three hours at 105°C, then kept in a closed container and allowed to equilibrate to the ambient laboratory temperature. Care shall be taken to test the sample material quickly, i.e. typically in no more than 45 minutes after removal from the closed container in order to minimize moisture gain in the controlled laboratory environment.

The Rheological Creep Test and the Dynamical Mechanical Analysis (DMA) - Temperature Sweep Test mentioned hereinabove for measuring the cohesive strength parameter γ and the cross-over temperature parameter Tx respectively, are as described in the copending patent application EP 1447067, assigned to the Procter & Gamble Company.

### Alternative sample preparation for all tests herein when starting from an absorbent article.

When starting from an article comprising the absorbent polymer material and the inert material, the two materials can be isolated with known means, typically from the layer of thermoplastic material and the substrate layer, in order to be tested. Typically, in a disposable absorbent article the topsheet can be removed from the backsheet and the absorbent core can be separated from any additional layers, comprising the optional cover layer, if present. The absorbent polymer material and the inert material can be removed from the substrate layer and the layer of thermoplastic material, e.g. mechanically if possible, or by use of a suitable solvent, in case e.g. the thermoplastic material is a hot melt adhesive. Particles of absorbent polymer material and inert material can be hence isolated from other elements of the core e.g. by washing with a suitable solvent which does not interact with the absorbent polymer material and the inert material, as can be readily determined by the man skilled in the art. The solvent is then let to evaporate and the absorbent polymer material and the inert material can be separated from each other with known means, and collected in the necessary amounts to run the tests. Alternatively, the average particle size of the absorbent polymer material and of the inert material can be measured for example with optical means, e.g. based on light scattering and image analysis techniques, directly on the structure typically comprising the substrate layer, the absorbent polymer material, the inert material, and the layer of thermoplastic material, wherein a comparison between the average particle size of the two materials is possible, as can be readily determined by the skilled person.

### Artificial Menstrual Fluid (AMF)

Artificial Menstrual Fluid is based on modified sheep's blood that has been modified to ensure it closely resembles human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent core (28) for an absorbent article (20) intended for absorption of menses or blood or vaginal discharges, said core (28) comprising a substrate layer (100),
said substrate layer (100) comprising a first surface and a second surface,
said absorbent core (28) further comprising a non uniform layer of absorbent polymer material (110),
said non uniform layer of absorbent polymer material (110) comprising a first surface and a second surface,
said absorbent core (28) further comprising a layer of a thermoplastic material (120), said layer of thermoplastic material (120) comprising a first surface and a second surface,
wherein said second surface of said non uniform layer of absorbent polymer material (110) is in at least partial contact with said first surface of said substrate layer (100), and wherein portions of said second surface of said layer of thermoplastic material (120) are in direct contact with said first surface of said substrate layer (100) and portions of said second surface of said layer of thermoplastic material (120) are in direct contact with said first surface of said non uniform layer of absorbent polymer material (110), said substrate layer (100) comprising a fibrous web of fibres,
wherein
said absorbent core (28) comprises an inert material (150),
said absorbent core being **characterized in that** said inert material (150) is comprised within said non uniform layer of absorbent polymer material (110), and
said absorbent polymer material (110) and said inert material (150) are in particulate form, and wherein said layer of thermoplastic material (120) is a layer of fiberized hot-melt adhesive.

2. An absorbent core (28) according to claim 1, wherein said absorbent polymer material (110) and said inert material (150) both have an average particle size from 200 µ to 600 µ, preferably from 300 µ to 500 µ.

3. An absorbent core (28) according to claims 1 or 2, wherein said inert material (150) has a bulk density which is from 70% to 120%, preferably from 80% to 110% the bulk density of said absorbent polymer material.

4. An absorbent article (28) according to any preceding claim, wherein said inert material (150) is silica.

5. An absorbent core (28) according to any preceding claim, wherein said absorbent polymer material (110) is comprised in a basis weight of less than 250 g/m², preferably of less than 220 g/m², more preferably from 60 g/m² to 180g/m², even more preferably from 100 g/m² to 160 g/m², by weight of said absorbent polymer material (110) per square meter of the zone of application.

6. An absorbent core (28) according to any preceding claim, wherein said inert material (150) is comprised in an amount from 10% to 100%, preferably from 20% to 70%, more preferably from 30% to 60% by weight of the amount of said absorbent polymer material (110).

7. An absorbent core (28) according to any preceding claim, further comprising a cover layer (130) comprising a first surface and a second surface, and wherein said second surface of said cover layer (130) is in direct contact with said first surface of said layer of thermoplastic material (110).

8. An absorbent article (20) comprising a liquid permeable topsheet (30), a backsheet (40), and an absorbent core (28) according to any preceding claim comprised therebetween.

## Patentansprüche

1. Absorptionskern (28) für einen Absorptionsartikel (20), der zur Absorption von Menstruationsflüssigkeit oder Blut oder Vaginalausfluss vorgesehen ist, wobei der Kern (28) eine Substratschicht (100) umfasst, wobei die Substratschicht (100) eine erste Oberfläche und eine zweite Oberfläche umfasst,
wobei der Absorptionskern (28) ferner eine nicht gleichförmige Schicht aus absorbierendem Polymermaterial (110) umfasst,
wobei die nicht gleichförmige Schicht aus absorbierendem Polymermaterial (110) eine erste Oberfläche und eine zweite Oberfläche umfasst,
wobei der Absorptionskern (28) ferner eine Schicht aus einem thermoplastischen Material (120) umfasst,
wobei die Schicht aus thermoplastischem Material (120) eine erste Oberfläche und eine zweite Oberfläche umfasst,
wobei die zweite Oberfläche der nicht gleichförmigen Schicht aus absorbierendem Polymermaterial (110) wenigstens teilweise mit der ersten Oberfläche der Substratschicht (100) in Kontakt steht, und wobei Abschnitte der zweiten Oberfläche der Schicht aus thermoplastischem Material (120) in direktem Kontakt mit der ersten Oberfläche der Substratschicht (100) stehen und Abschnitte der zweiten Oberfläche der Schicht aus thermoplastischem Material (120) in direktem Kontakt mit der ersten Oberfläche der nicht gleichförmigen Schicht aus absorbierendem Polymermaterial (110) stehen, wobei die Substratschicht (100) eine Faserbahn von Fasern umfasst,
wobei
der Absorptionskern (28) ein Inertmaterial (150) umfasst,
wobei der Absorptionskern **dadurch gekennzeichnet ist, dass** das Inertmaterial (150) innerhalb der nicht gleichförmigen Schicht aus absorbierendem Polymermaterial (110) enthalten ist und
das absorbierende Polymermaterial (110) und das Inertmaterial (150) in Teilchenform vorliegen, und wobei die Schicht aus thermoplastischem Material (120) eine Schicht aus zerfasertem Heißschmelzkleber ist.

2. Absorptionskern (28) nach Anspruch 1, wobei das absorbierende Polymermaterial (110) und das Inertmaterial (150) beide eine durchschnittliche Teilchengröße von 200 µ bis 600 µ, vorzugsweise von 300 µ bis 500 µ aufweisen.

3. Absorptionskern (28) nach Anspruch 1 oder 2, wobei das Inertmaterial (150) eine Rohdichte aufweist, die 70 % bis 120 %, vorzugsweise 80 % bis 110 % der Rohdichte des absorbierenden Polymermaterials beträgt.

4. Absorptionsartikel (28) nach einem der vorstehenden Ansprüche, wobei das Inertmaterial (150) Siliciumdioxid ist.

5. Absorptionskern (28) nach einem der vorstehenden Ansprüche, wobei das absorbierende Polymermaterial (110) in einem Basisgewicht von weniger als 250 g/m², vorzugsweise von weniger als 220 g/m², mehr bevorzugt von 60 g/m² bis 180 g/m², noch mehr bevorzugt von 100 g/m² bis 160 g/m², bezogen auf das Gewicht des absorbierenden Polymermaterials (110) pro Quadratmeter des Anwendungsbereichs, enthalten ist.

6. Absorptionskern (28) nach einem der vorstehenden Ansprüche, wobei das Inertmaterial (150) in einer Menge von 10 % bis 100 %, vorzugsweise von 20 % bis 70 %, mehr bevorzugt von 30 % bis 60 %, bezogen auf das Gewicht der Menge des absorbierenden Polymermaterials (110), enthalten ist.

7. Absorptionskern (28) nach einem der vorstehenden Ansprüche, ferner umfassend eine Deckschicht (130), die eine erste Oberfläche und eine zweite Oberfläche umfasst, und wobei die zweite Oberfläche der Deckschicht (130) in direktem Kontakt mit der ersten Oberfläche der Schicht aus thermoplastischem Material (110) steht.

8. Absorptionsartikel (20), umfassend eine flüssigkeitsdurchlässige Oberschicht (30), eine Unterschicht (40) und einen Absorptionskern (28) nach einem der vorstehenden Ansprüche, der dazwischen enthalten ist.

## Revendications

1. Âme absorbante (28) pour un article absorbant (20) destiné à l'absorption des règles ou de sang ou d'écoulements vaginaux, ladite âme (28) comprenant une couche (100) de substrat, ladite couche (100) de substrat comprenant une première surface et une deuxième surface,
ladite âme absorbante (28) comprenant en outre une couche non uniforme de matériau polymère absorbant (110),
ladite couche non uniforme de matériau polymère absorbant (110) comprenant une première surface et une deuxième surface,
ladite âme absorbante (28) comprenant en outre une couche de matériau thermoplastique (120),
ladite couche de matériau thermoplastique (120) comprenant une première surface et une deuxième surface,
dans laquelle ladite deuxième surface de ladite couche non uniforme de matériau polymère absorbant (110) est en contact au moins partiel avec ladite première surface de ladite couche de substrat (100) et dans laquelle des parties de ladite deuxième surface de ladite couche de matériau thermoplastique (120) sont en contact direct avec ladite première surface de ladite couche de substrat (100) et des parties de ladite deuxième surface de ladite couche de matériau thermoplastique (120) sont en contact direct avec ladite première surface de ladite couche non uniforme de matériau polymère absorbant (110), ladite couche de substrat (100) comprenant une nappe de fibres fibreuse,
dans laquelle :
ladite âme absorbante (28) comprend un matériau inerte (150),
ladite âme absorbante (28) étant **caractérisée en ce que** ledit matériau inerte (150) se trouve dans ladite couche non uniforme de matériau polymère absorbant (110) et
ledit matériau polymère absorbant (110) et ledit matériau inerte (150) sont sous forme particulaire, et dans lequel ladite couche de matériau thermoplastique (120) est une couche d'adhésif thermofusible défibrée.

2. Âme absorbante (28) selon la revendication 1, dans laquelle ledit matériau polymère absorbant (110) et ledit matériau inerte (150) ont tous les deux une taille de particule moyenne allant de 200 µ à 600 µ, de préférence de 300 µ à 500 µ.

3. Âme absorbante (28) selon les revendications 1 ou 2, dans laquelle le matériau inerte (150) a une densité en vrac qui va de 70 % à 120 %, de préférence de 80 % à 110 % de la densité en vrac dudit matériau polymère absorbant.

4. Article absorbant (28) selon l'une quelconque des revendications précédentes, dans lequel ledit matériau inerte (120) est de la silice.

5. Âme absorbante (28) selon l'une quelconque des revendications précédentes, dans laquelle ledit matériau polymère absorbant (110) est compris dans une masse surfacique inférieure à 250 g/m², de préférence inférieure à 220 g/m², plus préférablement de 60 g/m² à 180 g/m², plus préférablement encore de 100 g/m² à 160 g/m², en poids dudit matériau polymère absorbant (110) par mètre carré de la zone d'application.

6. Âme absorbante (28) selon l'une quelconque des revendications précédentes, dans laquelle ledit matériau inerte (150) est compris dans une quantité allant de 10 % à 100 %, de préférence de 20 % à 70 %, plus préférablement de 30 % à 60 % en poids de la quantité dudit matériau polymère absorbant (110).

7. Âme absorbante (28) selon l'une quelconque des revendications précédentes, comprenant en outre une couche de recouvrement (130) comprenant une première surface et une deuxième surface, et dans laquelle ladite deuxième surface de ladite couche de recouvrement (130) est en contact direct avec ladite première surface de ladite couche de matériau thermoplastique (110).

8. Article absorbant (20) comprenant une feuille supérieure perméable aux liquides (30), une feuille de fond (40) et une âme absorbante (28) selon l'une quelconque des revendications précédentes se trouvant entre celles-ci.
